# EUROPÄISCHE PATENTANMELDUNG

(11) **EP 3 798 206 A1**
(43) Veröffentlichungstag der Anmeldung: **31.03.2021**
(21) Anmeldenummer: 19199483.9
(22) Anmeldetag: 25.09.2019
(51) Int. Cl.: C07C 67/39, C07C 69/54, C07C 67/54

(54) **VERFAHREN ZUR HERSTELLUNG VON ALKYLMETHACRYLATEN MIT VERBESSERTER WASSER- UND SÄUREFÜHRUNG**

(71) Anmelder: Röhm GmbH, 64295 Darmstadt (DE)
(72) Erfinder: Die Erfindernennung liegt noch nicht vor
(74) Vertreter: Röhm Patent Association

(57) **Zusammenfassung**

Die vorliegende Erfindung betrifft ein Verfahren zur Herstellung von Alkylmethacrylaten, insbesondere Methylmethacrylat (MMA), umfassend in einer ersten Reaktionsstufe die Herstellung von Methacrolein (MAL), in einer zweiten Reaktionsstufe die direkte oxidative Veresterung (DOE) des Methacroleins mit einem Alkohol, bevorzugt Methanol, zu einem Alkylmethacrylat, und die Aufarbeitung des Alkylmethacrylat-Rohproduktes aus der zweiten Reaktionsstufe. Insbesondere betrifft die vorliegende Erfindung eine optimierte Aufarbeitung des Reaktoraustrags der oxidativen Veresterung von Methacrolein, wobei die Menge an eingesetztem Wasser, die Menge an eingesetzter Säure und/oder die Menge an wässrigen Abfallströmen durch eine optimierte Rückführung der anfallenden Prozesswasser-Ströme minimiert werden.

## Beschreibung

### Gebiet der Erfindung

Die vorliegende Erfindung betrifft ein Verfahren zur Herstellung von Alkylmethacrylaten, insbesondere Methylmethacrylat (MMA), umfassend in einer ersten Reaktionsstufe die Herstellung von Methacrolein (MAL), in einer zweiten Reaktionsstufe die direkte oxidative Veresterung (DOE) des Methacroleins mit einem Alkohol, bevorzugt Methanol, zu einem Alkylmethacrylat, und die Aufarbeitung des Alkylmethacrylat-Rohprodukts aus der zweiten Reaktionsstufe.

Insbesondere betrifft die vorliegende Erfindung eine optimierte Aufarbeitung des Reaktoraustrags der oxidativen Veresterung von Methacrolein, wobei die Menge an eingesetztem Wasser, die Menge an eingesetzter Säure und/oder die Menge an wässrigen Abfallströmen durch eine optimierte Rückführung der anfallenden Prozesswasser-Ströme minimiert werden.

### Stand der Technik

Methylmethacrylat wird in großen Mengen zur Herstellung von Polymeren und Copolymeren mit anderen polymerisierbaren Verbindungen eingesetzt. Weiterhin ist Methylmethacrylat (MMA) ein wichtiger Baustein für diverse, auf Methacrylsäure (MAS) basierende Spezialester, die durch Veresterung der Säure bzw. Umesterung von MMA mit dem entsprechenden Alkohol hergestellt werden können. Hieraus ergibt sich ein großes Interesse an möglichst einfachen, wirtschaftlichen und umweltschonenden Herstellungsverfahren für diesen Ausgangsstoff.

Methylmethacrylat (MMA) wird heute mittels diverser Verfahren, die von C₂-, C₃- oder C₄-Bausteinen ausgehen, hergestellt. In einem dieser Verfahren wird MMA durch Oxidation von Isobutylen oder *tert-*Butanol mit Luftsauerstoff in der Gasphase an einem heterogenen Kontakt zu Methacrolein (MAL) und anschließender oxidativer Veresterungsreaktion von Methacrolein unter Verwendung von Methanol erhalten. Dieses von ASAHI entwickelte Verfahren ist unter anderem in den Dokumenten US 5,969,178 und US 7,012,039 beschrieben. Nachteil dieses Verfahrens ist insbesondere ein sehr hoher Energiebedarf.

Die Herstellung von MMA nach dem sogenannten Asahi Verfahren erfolgt ausgehend von C4 (Isobuten oder tert-Butanol), mit Zwischenisolierung von MAL und anschließender direkter oxidativer Veresterung (abgekürzt "DOE") des MAL mit Methanol zu MMA, wobei Methacrylsäure (MAS) durch Reaktion mit Wasser als Nebenprodukt gebildet wird.

In einer Weiterentwicklung des Verfahrens wird das Methacrolein in der ersten Stufe aus Propanal und Formalin gewonnen. Ein solches Verfahren ist in WO 2014/170223 beschrieben.

Im folgenden Schema 1 ist die Reaktionmatrix für die Herstellung von MMA wiedergegeben, wobei exemplarisch die Herstellung von Methacrolein ausgehend von Ethylen, Synthesegas und Formaldehyd beschrieben ist. Wie oben ausgeführt ist auch der Methacrolein Zugang ausgehend von Isobuten oder tert-Butanol möglich.

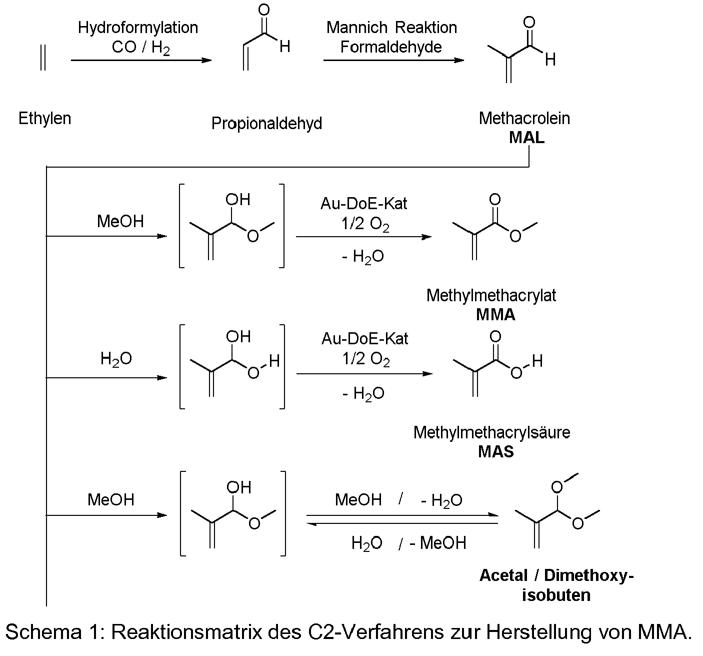

Methacrylsäure bildet sich bei der oxidativen Veresterung (DOE Reaktion) in Gegenwart von Wasser, welches üblicherweise bei der kontinuierlichen Durchführung der Reaktion in einer stationären Konzentration zwischen 2 und 20 Gew% im Reaktor der oxidativen Veresterung anwesend ist. Führt man die DOE Reaktion bei konstantem pH durch, so wird die sich bildende Methacrylsäure zumindest anteilig mit alkalischen bzw. basischen Hilfsstoffen, im einfachsten Fall Alkaliverbindungen neutralisiert.

Das Rohprodukt der oxidativen Veresterung enthält üblicherweise das Alkylmethacrylat als Hauptreaktionsprodukt, nicht umgesetzte Edukte, insbesondere Methacrolein und Alkohol, geringe Mengen an Wasser sowie verschiedene Nebenprodukte, wie Methacrylsäure (MAS) und deren Salze (z.B. Natriummethacrylat), Acetale (z.B. Dimethoxyisobuten DMIB), sowie weitere schwersiedende Komponenten wie z.B. Additionsprodukte und Diels-Alder-Produkte, z.B. Hydroxyisobuttersäure und deren korrespondierendee Ester, dimeres Methacrolein (DIMAL) und deren Ester (DIMAL Ester). Oftmals müssen die schwersiedenden Nebenprodukte abgetrennt werden, um das gewünschte Alkylmethacrylat-Produkt (z.B. MMA) in einer geeigneten Reinheit für die Polymerisation, typischerweise von deutlich größer als 99 Gew%, zu erhalten.

In US 5,969,178 und dem dort angeführten Stand der Technik ist ein Verfahrensind Verfahrensvarianten zur oxidativen Umsetzung von Isobuten oder tert-Butanol zu Methacrolein und die folgende oxidative Veresterung zu MMA beschrieben. Aus dem Rohprodukt der oxidativen Veresterung wird in einer ersten Destillationsstufe ein Gemisch aus Methacrolein und Methanol unterhalb des Kopfes der Kolonne abgetrennt, während über Kopf leichtsiedende Bestandteile entfernt werden. Der MMA-haltige Sumpf wird darauf in eine zweite Destillationsstufe zusammen mit einem ungesättigten Kohlenwasserstoff geführt, in der über Kopf ein Azeotrop aus Methanol und gesättigten Kohlenwasserstoffen abgetrennt wird. Der Sumpf, enthaltend das Roh-MMA wird einer weiteren Aufarbeitung zugeführt, während aus der über Kopf gewonnen Fraktion mittels eines Phasentrenners und einer dritten Destillationskolonne Methanol isoliert und zurück in den Reaktor geführt wird. Dabei ist zu berücksichtigen, dass das Methanol aufgrund des gebildeten Azeotrops relativ viel Wasser enthalten kann und damit einer Entwässerung zugeführt werden muss.

Als Alternative zu diesem Verfahren offenbart US 5,969,178 die Aufarbeitung in nur einer Kolonne, wobei sich bei dieser der Zulauf zwingend oberhalb des Sumpfes befindet. Über Kopf werden in dieser Kolonne leichtsiedende Bestandteile aus dem Reaktoraustrag entfernt. Im Sumpf verbleibt eine Mischung aus Roh-MMA und Wasser, welche einer weiteren Aufarbeitung zuzuführen ist. Über einen Seitenstrom wird der Kolonne schließlich eine Mischung aus Methacrolein und Methanol, zur Zurückführung in den Reaktor, entnommen. Im Dokument US 5,969,178 wird darauf hingewiesen, dass ein solches Verfahren aufgrund diverser Azeotrope schwierig durchzuführen ist. Die US 5,969,178 bietet keine Lösung an für die Abtrennung von MMA von Methacrylatsalzhaltigen wässrigen Lösungen, die zwangsläufig bei der beschriebenen Verfahrensweise anfallen.

In US 7,012,039 wird eine etwas abweichende Aufarbeitung des Reaktoraustrags der oxidativen Veresterung offenbart. Die Verwendung eines bleihaltigen Katalysators, der offensichtlich auch Blei in die Reaktionslösung abgibt, macht insbesondere die nachfolgende Aufarbeitung komplexer, da unslösliche Bleisalze im Verfahren entstehen und diese gesondert mit hohem Aufwand abgetrennt werden müssen. Hier wird in einer ersten Destillationsstufe über Siebböden Methacrolein über Kopf abdestilliert und die wässrige, MMA haltige Mischung aus dem Sumpf in einen Phasenseparator geleitet. In diesem wird die Mischung durch Zugabe von Schwefelsäure auf einen pH-Wert von ca. 2 eingestellt. Die Trennung des schwefelsauren Wassers von der organischen bzw. Öl-Phase erfolgt dann mittels Zentrifugieren. Diese organische Phase wird in einer weiteren Destillation in hochsiedende Bestandteile und eine MMA haltige Phase, welche über Kopf abgezogen wird, getrennt.

Die MMA haltige Phase wird danach in einer dritten Destillation von niedrigsiedenden Bestandteilen getrennt. Darauf folgt noch eine vierte Destillation zur endgültigen Reinigung.

Problematisch an den Verfahren gemäß US 7,012,039 ist die Schwefelsäure, die in großen Mengen zugegeben werden muss und in Teilen der Anlage korrosiv wirken kann. Entsprechend müssen diese Teile, wie insbesondere der Phasenseparator oder auch die zweite Destillationskolonne aus dafür geeigneten Materialien gefertigt sein. Gemäß dieser Veröffentlichung ist keine dem Fachmann ersichtliche Kreislaufführung von säurehaltigen wässrigen Strömen ableitbar. Weiterhin bietet das Verfahren nach US 7,012,039 keine Möglichkeiten Methacrylsäure oder das im Produkt verbleibende restliche Methanol wiederzugewinnen.

WO 2014/170223 beschreibt ein ähnliches Verfahren wie US 7,012,039. Es besteht der Unterschied, dass der pH-Wert in der oxidativen Veresterung mittels Zugabe einer methanolischen Natriumhydroxidlösung in einer Kreislaufführung eingestellt wird. Die pH Regulierung dient unter anderem dazu, den Katalysator zu schonen. Weiterhin ist die Abtrennung der wässrigen Phase in der Phasenseparation aufgrund des Salzgehalts einfacher. Allerdings führt dies auch dazu, dass die entstehende Methacrylsäure als Natriumsalz vorliegt und später mit der wässrigen Phase abgetrennt und verworfen wird. Bei der Variante einer Schwefelsäurezugabe in der Phasenseparation wird die freie Säure zwar wiedergewonnen, aber auf Kosten von anfallendem Natrium(hydrogen)sulfat, welches bei der Entsorgung zu anderen Problemen führen kann.

WO 2017/046110 beschreibt ein Verfahren zur Herstellung von MMA, wobei das Rohprodukts der oxidativen Veresterung aufgearbeitet wird durch Destillation der wässrigen Phase einer Extraktion, welche Methanol, mindestens ein Alkalisalz, Methacylsäure und eine starke anorganische Säure enthält. Die niedrigsiedende Fraktion dieser Destillationsstufe, welche hauptsächlich Methanol enthält, wird in die oxidative Veresterung zurückgeführt. Die wässrige Sumpf-Fraktion wird ausgeschleust und entsorgt. Ein Seitenstrom der Destillation, enthaltend Wasser und Methacylsäure, kann der Extraktion zugeführt werden. Durch die Zugabe einer starken Säure bei der Extraktion des Rohproduktes werden Salze der Methacylsäure neutralisiert, wobei die freie Methacylsäure günstig gewonnen werden kann.

Das Dokument WO 2019/042807 beschreibt ein Verfahren zur Herstellung von PMMA-Harzen, wobei zunächst die Herstellung des Monomeren erfolgt. Das MMA-Rohprodukt wird nach der oxidativen Veresterung in einem Reaktor durch Zugabe einer organischen und/oder mineralischen Säure nachbehandelt, um das Nebenprodukt Dimethoxyisobuten hydrolytisch zu spalten.

An den Verfahren des Standes der Technik zur Aufarbeitung des Rohproduktes der oxidativen Veresterung ist nachteilig, das relative hohen Mengen an Säure und Wasser an verschiedenen Stellen dem Verfahren zugeführt werden und somit auch ein hoher Anteil an wässrigen Abfallströmen anfällt, welche mit Säure und/oder organischen Komponenten belastet sind und entsorgt werden müssen.

Zudem erfordert die Zugabe von starken Säuren im entsprechenden Verfahrensteil einen erhöhten apparativen Aufwand, wie insbesondere die Ausgestaltung in korrosionsbeständigen Materialen.

### Aufgabe

In Anbetracht des Standes der Technik ist es daher Aufgabe der vorliegenden Erfindung, ein technisch verbessertes Verfahren zur oxidativen Veresterung von Methacrolein zur Verfügung zu stellen, das nicht mit den oben beschriebenen Nachteilen herkömmlicher Verfahren behaftet ist. Insbesondere ist es Aufgabe der vorliegenden Erfindung, eine verbesserte Aufarbeitung des Rohproduktes der oxidativen Veresterung von Methacrolein bereitzustellen, wobei die Menge an eingesetztem Wasser und an eingesetzter Säure sowie die Menge an wässrigen Abfallströmen vermindert werden kann. Insbesondere soll ein Verfahren zur Verfügung gestellt werden, das mit einem möglichst geringen Entsorgungsaufwand, insbesondere durch ein reduziertes Anfallen wässriger und organischer Bestandteile und Säuren im Abfallstrom, betrieben werden kann.

Weiterhin soll das Verfahren gegenüber dem Stand der Technik kostengünstig sein, beispielsweise in Bezug auf die bei der Konstruktion der Anlage zu verwendenden Materialien. Weiterhin sollte das Verfahren eine möglichst hohe Ausbeute an Alkylmethacrylat und die Gewinnung von methacrylischen Nebenprodukten, wie Methacylsäure, ermöglichen. Grundsätzlich sollte es das Verfahren ermöglichen einen möglichst hohen Anteil von Nebenprodukten umzuwandeln und zurückzuführen.

### Lösung der Aufgabe

Es wurde überraschenderweise gefunden, dass die Zufuhr von Säure und Wasser sowie die Menge an wässrigem Abwasser durch eine optimierte Führung der anfallenden Prozesswasser-Ströme minimiert werden kann. Insbesondere wurde gefunden, dass durch eine optimierte und gezielte Verteilung des wässrigen Sumpfes der Destillationsstufe zur Rückgewinnung des Alkohols eine Einsparung an eingesetztem Wasser und/oder eingesetzter Säure sowie eine Verminderung des Abwasserstromes, der dem Verfahren entnommen werden muss, erzielt werden kann.

Die oben beschriebenen Aufgaben werden erfindungsgemäß gelöst durch ein Verfahren zur Herstellung von Alkylmethacrylaten, bei dem in einer ersten Reaktionsstufe in einem Reaktor I Methacrolein (MAL) hergestellt und dieses in einer zweiten Reaktionsstufe in einem Reaktor II oxidativ mit einem Alkohol, bevorzugt Methanol, in Gegenwart eines sauerstoffhaltigen Gases unter Entstehung von Reaktionswasser in flüssiger Phase zu einem Alkylmethacrylat, bevorzugt zu Methylmethacrylat (MMA), verestert wird, dadurch gekennzeichnet dass
a. die Aufarbeitung der Reaktionsmischung aus Reaktor II zu Alkylmethacrylat mindestens eine Destillation und mindestens eine Extraktion umfasst;
b. eine wässrige, Alkohol und Alkali- und/oder Erdalkalisalz einer Brönstedt-Säure enthaltende Phase der Extraktion in mindestens einer Destillation in einer Kolonne II (Destillationsstufe II) derart behandelt wird, dass im Sumpf der Kolonne II ein Prozesswasserstrom (Sumpf-Fraktion W4) enthaltend Reaktionswasser und Alkali- und/oder Erdalkalisalz einer Brönstedt-Säure entsteht, wobei in diesem Prozesswasserstrom der Gehalt an Alkohol und Alkylmethacrylat kleiner 5 Gew.-%, bezogen auf den gesamten Prozesswasserstrom, beträgt, und
c. dieser Prozesswasserstrom aus dem Sumpf der Kolonne II (Sumpf-Fraktion W4) teilweise aus dem Verfahren ausgeschleust und einer Entsorgung zugeführt wird und teilweise in die Aufarbeitung der Reaktionsmischung aus Reaktor II zurückgeführt wird.

### Beschreibung der Erfindung

Der Ausdruck Strom, Phase oder Fraktion enthaltend ein Edukt, Produkt und/oder Nebenprodukt des beanspruchten Verfahrens ist im Sinne der Erfindung so zu verstehen, dass die genannten Verbindungen in dem jeweiligen Strom zu finden ist, beispielsweise ist der überwiegende Anteil des Edukts, Produkts und/oder Nebenprodukts in dem entsprechenden Strom zu finden. Grundsätzlich können neben den genannten Verbindungen weitere Bestandteile enthalten sein. Oftmals dient die Nennung der Bestandteile der Verdeutlichung des jeweiligen Verfahrensschritts.

Bevorzugt wird die mindestens eine Destillation des erfindungsgemäßen Verfahrens, beispielsweise die im Folgenden beschriebenen Destillationsstufen I; II, III, und IV, in Destillationskolonnen durchgeführt. Die Destillationskolonnen werden entsprechend der Destillationsstufe mit I; II, III, IV etc. bezeichnet. Dem Fachmann sind typische Ausführungsformen von Destillationskolonnen bekannt. Typischerweise können Bodenkolonne, die beispielsweise mit Sieböden, Kaskadenböden, Turbogridböden und/oder Riffelböden ausgestattet sind, oder gepackte Kolonnen, wie beispielsweise Füllkörperkolonnen (z.B. mit Raschig Superring von Raschig) oder Kolonnen mit regelmäßigem Packmaterial (z.B. Mellapak von Sulzer), eingesetzt werden. Typischerweise werden bei den Destillationsstufen mindestens eine schwersiedende Sumpf-Fraktion und mindestens eine leichtsiedende Kopf-Fraktion erhalten. Die Destillationstemperatur in den verschiedenen Destillationsstufen wird von Fachmann in Abhängigkeit vom Destillationsdruck, der Zusammensetzung der zu trennenden Mischung, der Anzahl der Böden und Ausgestaltung der Destillationskolonne sowie weiteren Faktoren gewählt. Vorzugsweise liegt die Destillationstemperatur im Bereich von 20 bis 120 °C

Bevorzugt handelt es sich bei dem Alkohol um Methanol und bei dem Alkylmethacrylat um Methylmethacrylat (MMA).

Bevorzugt handelt es sich bei der Brönstedt-Säure (im Folgenden auch als Säure S bezeichnet) um eine starke Säure, insbesondere eine Säure mit einem pK_{S}-Wert, der kleiner ist als der pK_{S}-Wert von Methacrylsäure. Bevorzugt weist die Brönstedt-Säure einen pK_{S}-Wert von kleiner 3, besonders bevorzugt von kleiner 2 auf. Es können starke anorganische Säuren, wie Schwefelsäure oder Phosphorsäure, oder starke organische Säuren, wie Methansulfonsäure oder Toluolsulfonsäure, eingesetzt werden. Bevorzugt handelt es sich bei der Brönstedt-Säure um Schwefelsäure.

Bevorzugt wird das erfindungsgemäße Verfahren kontinuierlich oder halbkontinuierlich betrieben. Bevorzugt wird das Verfahren, mit Ausnahme von diskontinuierlichen Ausschleusungen von Abfallströmen, kontinuierlich betrieben.

Bevorzugt wird der Prozesswasserstrom aus dem Sumpf der Kolonne II teilweise in die Extraktion zurückgeführt, wobei dieser zurückgeführte Prozesswasserstrom in der Extraktion mit einer Alkylmethacrylat und Alkohol enthaltenden organischen Phase (insbesondere organische Phase P1) in Kontakt gebracht wird.

Bevorzugt handelt es sich bei dem Prozesswasserstrom aus dem Sumpf der Kolonne II um die Sumpf-Fraktion W4 des unten beschriebenen bevorzugten Verfahrens.

Bevorzugt wird der Prozesswasserstrom aus dem Sumpf der Kolonne II teilweise in einen Reaktor III (insbesondere Acetal-Spalter III wobei in Strom W1 enthaltende Acetale gespalten werden) zurückgeführt, wobei dieser zurückgeführte Prozesswasserstrom in Reaktor III mit einer Alkylmethacrylat und Alkohol enthaltenden organischen Phase (insbesondere Sumpf-Fraktion W1), welche einen Gehalt an Methacrolein-Acetal (insbesondere von Dimethoxyisobuten) von kleiner 3 Gew.-%, bezogen auf die organischen Phase, aufweist, in Kontakt gebracht wird.

Bevorzugt wird die Extraktion in einer Extraktionskolonne und/oder in einer in Reihe geschalteten Serie von mindestens zwei Mixer-Settler-Apparaten durchgeführt und der Prozesswasserstrom aus dem Sumpf der Kolonne II wird unterhalb des Kopfes der Extraktionskolonne oder im Mixerbereich eines Mixer-Settler-Apparates zugegeben, und optional wird im Kopfbereich der Extraktionskolonne Wasser, insbesondere vollentsalztes Wasser, zugegeben.

Bei dem Wasser, das dem erfindungsgemäßen Verfahren zugeführt wird, handelt es sich insbesondere um vollentsalztes Wasser.

Bevorzugt erfolgt die Zugabe von Wasser und optional einer Brönstedt-Säure zu der Extraktion oberhalb von der Zugabe des Prozesswasserstroms aus dem Sumpf der Kolonne II.

Bevorzugt erfolgt in Reaktor III die Zugabe einer Brönstedt-Säure und diese Zugabe wird so gewählt, dass sich im kontinuierlichen Betrieb ein pH Wert im Bereich von 1,5 bis 2,5 in dem Prozesswasserstrom aus dem Sumpf der Kolonne II (insbesondere der Sumpf-Fraktion W4) einstellt.

In einer bevorzugten Ausführungsform erfolgt die Zugabe einer Brönstedt-Säure in das erfindungsgemäße Verfahren ausschließlich über die Zugabe in Reaktor III. Es ist auch denkbar die Säurezugabe an verschiedenen Stellen des Verfahrens durchzuführen, um die unterschiedlichen pH Werte der unterschiedlichen Verfahrensoperationen genauer einstellen zu können.

Bevorzugt wird in der Extraktion eine organische Phase enthaltend Alkylmethacrylat und Methacrylsäure erhalten und die organische Phase aus der Extraktion (insbesondere die organische Phase P1 oder die Kopf-Fraktion W5 aus Destillationsstufe III) wird in einer Destillationsstufe IV (Kolonne IV) in eine Sumpf-Fraktion enthaltend Alkylmethacrylat (insbesondere Sumpf-Fraktion W8) und eine leichter-siedende Kopf-Fraktion (insbesondere Kopf-Fraktion W7) getrennt.

Bevorzugt wird die Fraktion aus der Kolonne IV mit Wasser versetzt und danach in einem Phasentrenner I in eine organische Phase (insbesondere organische Phase P3) und in eine wässrige Phase (insbesondere wässrige Phase P4) aufgetrennt.

Bevorzugt wird die wässrige Phase aus dem Phasentrenner I (insbesondere wässrige Phase P4) mit mindestens einer Brönstedt-Säure in einem Reaktor IV vermischt, wobei in der wässrige Phase aus dem Phasentrenner I enthaltende Ester-Nebenprodukte gespalten werden und Alkohol zurückgewonnen wird, und wobei optional der Prozesswasserstrom aus dem Sumpf der Kolonne II teilweise in den Reaktor IV geführt wird.

Bevorzugt wird das Produkt aus dem Reaktor IV vollständig oder teilweise in die Kolonne II geführt.

In einer alternativen Ausführungsform kann die Actetalhydrolyse (DMIB + Wasser -> MAL zu MeOH) in Reaktor III und die in Reaktor IV ablaufende partielle Esterhydrolyse in einem Reaktionsgefäß zusammengeführt werden.

Bevorzugt wird die wässrige Phase aus Phasentrenner I vollständig oder teilweise in die Kolonne II geführt.

Bevorzugt wird die Reaktionsmischung aus Reaktor II in einer Destillationsstufe I (Kolonne I) getrennt, wobei Methacrolein und teilweise Alkohol über die Kopf-Fraktion entfernt und zum Reaktor II zurückgeführt werden.

Bevorzugt wird Methacrolein vollständig oder teilweise in die Destillationsstufe I zugegeben und über die Kopf-Fraktion der Destillationsstufe I zum Reaktor II geführt.

Bevorzugt ist der Alkohol Methanol und das Alkylmethacrylat ist Methylmethacrylat. Bevorzugt ist die Brönstedt-Säure Schwefelsäure.

### Erste Reaktionsstufe (Herstellung MAL)

Das erfindungsgemäß Verfahren umfasst in einer ersten Reaktionsstufe die Herstellung von Methacrolein (MAL) in einem Reaktor I. Dabei ist erfindungsgemäß die erste Stufe des Verfahrens zur Synthese des Methacroleins frei wählbar. Die erste Reaktionsstufe des Verfahrens kann sowohl eine Erststufen-Synthese auf Basis von tert-Butanol oder Isobutylen, als auch eine Erststufen-Synthese auf Basis auf Basis von Propanal und Formalin umfassen.

Bei der Herstellung von Methacrolein (MAL) auf Basis von Propanal und Formalin kommen im Prinzip zwei Verfahrensvarianten in Frage, welche Methacrolein in einer Qualität liefern, das in der zweiten Reaktionsstufe (direkte oxidative Veresterung, DOE-Reaktion) eingesetzt werden kann. Zum einen können Propanal und Formalin in einem gerührten oder umgepumpten Reaktor bei Temperaturen von 20°C bis 120°C bei Drücken von 1 bar bis 10 bar umgesetzt werden. Dabei werden typischerweise Reaktionszeiten größer 10 min benötigt, um ausreichende Umsätze zu erzielen. Zum anderen können Propanal und Formalin zu MAL umgesetzt werden, wobei bei einem mittleren Druck zwischen 10 und 100 bar und bei höheren Temperaturen zwischen 120°C und 250°C die Reaktion mit einer Reaktionszeit von 2 Sekunden bis 20 Sekunden zu gewünschten, hohen Ausbeuten kommt.

Verfahren zur Herstellung von Methacrolein sind dem Fachmann bekannt und beispielsweise in Ullmanns Encyclopedia of Industrial Chemistry, 2012, Wiley-VCH Verlag GmbH, Weinheim (DOI: 10.1002/14356007.a01_149.pub2) beschrieben.

Es ist zudem möglich, dass es sich bei der ersten Reaktionsstufe um ein Verfahren ausgehend von C4-Rohstoffen handelt, insbesondere um die Oxidation von *tert*-Butanol und/oder Isobuten. Hierbei wird typischerweise Isobuten oder *tert*-Butanol in der Gasphase an einem heterogenen Kontakt mit sauerstoffhaltigen Gasen und bevorzugt mit Wasserdampf bei Temperaturen von über 300°C umgesetzt. Eine Vielzahl von Untervarianten und einsetzbarer Katalysatorsysteme sowie Isolierungsoptionen sind im einschlägigen Stand der Technik beschrieben. Eine Übersicht hierzu findet sich beispielsweise in "Trends and Future of Monomer-MMA Technologies", K. Nagai & T. Ui, Sumitomo Chemical Co., Ltd., Basic Chemicals Research Laboratory, 2005 (http://ww.sumitomo chem.co.jp/english/rd/ report/theses/docs/20040200_30a.pdf).

Bevorzugt handelt es sich bei der ersten Reaktionsstufe in Reaktor I um eine Umsetzung von Propanal mit Formalin. Die erste Reaktionsstufe kann optional eine Destillationskolonne zur Abtrennung von Leichtsiedern, wie verbliebenen Propanal, und/oder von Schwersiedern (z.B. dimeres Methacrolein), aufweisen.

### Zweite Reaktionsstufe (Direkte Oxidative Veresterung DOE)

Das erfindungsgemäß Verfahren umfasst in einer zweiten Reaktionsstufe die oxidative Veresterung (DOE-Reaktion) des Methacrolein mit einem Alkohol, bevorzugt Methanol, in einem Reaktor II, wobei ein Alkylmethacrylat, bevorzugt Methylmethacrylat (MMA), erhalten wird.

Bevorzugt wird die direkte oxidative Veresterung (DOE-Reaktion) in flüssiger Phase bei einem Druck von 2 bis 100 bar, bevorzugt bei einem Druck im Bereich von 2 bis 50 bar und einer Temperatur im Bereich von 10 bis 200 °C mit einem heterogenen Katalysator durchgeführt. Bei dem heterogenen Katalysator handelt es sich in der Regel um geträgerte, goldhaltige Nanopartikel mit einer Teilchengröße kleiner 20 nm, bevorzugt im Bereich von 0,2 bis 20 nm.

Typischerweise wird die direkte oxidative Veresterung unter Regulierung des pH-Wertes durchgeführt, um die optimale Aktivität des Katalysators zu gewährleisten, bevorzugt wird der pH-Wert auf einen pH von 6 -8, besonders bevorzugt auf pH 7 reguliert.

Bevorzugt umfasst die zweite Reaktionsstufe die Umsetzung von Methacrolein und einem Alkohol in Gegenwart eines sauerstoffhaltigen Gases bei moderaten Temperaturen zwischen 20 und 150 °C bei moderaten Drücken zwischen 1 und 20 bar in Gegenwart eines heterogenen partikulären Edelmetallhaltigen Katalysators, bevorzugt mit einer Partikelgröße von 1 bis 300 µm, wobei auch größere Partikel einsetzbar wären.

Im Stand der Technik ist eine Vielzahl von Katalysatoren für diese oxidative Veresterung von MAL mit Methanol zu MMA beschrieben. Das Dokument US 6,040,472 beschreibt Pd-Pb-haltige Katalysatoren auf einem oxidischen Träger; EP 1 393 800 beschreibt Gold-haltige Katalysatoren mit Goldpartikeln verteilt auf einem Siliziumoxid- bzw. einem TiO₂/SiO₂-Träger; EP 2 177 267 und EP 2 210 664 beschreiben nickelhaltige Katalysatoren mit Schalenstruktur; EP 2 210 664 offenbart einen Katalysator umfassend Nickeloxid und Goldnanopartikel auf einem Träger, WO 2017/084969 beschreibt Katalysatorsysteme auf Basis von mehreren Mischoxiden als Träger, die ebenfalls nanopartikuläres Gold neben Kobaltoxid als Aktivkomponente aufweisen.

Typischerweise umfasst der Reaktor II einen Zulauf von Metacrolein (MAL), einen Zulauf von Alkohol (insbesondere Methanol), eine Zuleitung von Sauerstoff- und/oder Luft-Zuleitung und einen Zulauf einer Base.

Optional können die Edukte oder ein Teil der Edukte, insbesondere Base und MeOH, vor Einleitung in den Reaktor II in einem optionalen Mischer gemischt werden.

Verfahren zur direkten oxidativen Veresterung von Methacrolein sind dem Fachmann bekannt. Weitere Einzelheiten der zweiten Reaktionsstufe sind beispielsweise in US 5,969,178, US 7,012,039, WO 2014/170223 und WO 2019/042807 beschrieben.

In einer bevorzugten Ausführungsform umfasst die Aufarbeitung der Reaktionsmischung aus Reaktor II die folgenden Schritte:
i. Trennen des Rohprodukts der oxidativen Veresterung aus Reaktor II in einer Destillationsstufe I, wobei Methacrolein und teilweise Alkohol in der Kopf-Fraktion entfernt und zum Reaktor II zurückgeführt werden, und wobei eine Sumpf-Fraktion W1, enthaltend Alkylmethacrylat, Methacrylsäure und/oder deren Salze, Alkohol und Wasser, erhalten wird;
ii. optional Einleiten der Sumpf-Fraktion W1 in einen Reaktor III und Zugabe mindestens einer Säure S, wobei in Strom W1 enthaltende Acetale gespalten werden, und wobei ein Strom W2 erhalten wird;
iii. Extraktion des Stroms W1 oder W2 mit Wasser und Auftrennen in eine organische Phase P1, enthaltend Alkylmethacrylat und Methacrylsäure, und eine wässrige Phase P2, enthaltend Wasser, Säure S und/oder deren Salze, Alkohol, Methacrylsäure und/oder deren Salze;
iv. Trennen der wässrigen Phase P2 aus der Extraktion in einer Destillationsstufe II, wobei eine Kopf-Fraktion W3, enthaltend hauptsächlich Alkohol, und eine Sumpf-Fraktion W4, enthaltend Wasser, Säure S und/oder deren Salze, und Methacrylsäure und/oder deren Salze, erhalten werden;
v. Trennen der organischen Phase P1 aus der Extraktion in einer Destillationsstufe III, wobei eine Kopf-Fraktion W5, enthaltend Alkylmethacrylat, und eine Sumpf-Fraktion W6, enthaltend Methacrylsäure, erhalten werden;
vi. Trennen der Kopf-Fraktion W5 aus Destillationsstufe III in einer Destillationsstufe IV, wobei eine Kopf-Fraktion W7 und eine Sumpf-Fraktion W8, enthaltend Alkylmethacrylat, erhalten werden;
vii. Vermischen der Kopf-Fraktion W7 aus der Destillationsstufe IV mit Wasser und Auftrennen in eine organische Phase P3 und eine wässrige Phase P4 in einem Phasentrenner I;
viii. optional Vermischen der wässrigen Phase P4 aus Phasentrenner I mit mindestens einer Säure S in Reaktor IV, wobei in P4 enthaltende Ester-Nebenprodukte gespalten werden und Alkohol zurückgewonnen wird, und wobei eine wässrige Phase P5 erhalten wird;
wobei die Sumpf-Fraktion W4 aus Destillationsstufe II vollständig oder teilweise in einen oder mehrere der Verfahrensteile, ausgewählt aus Reaktor III, Extraktion, Phasentrenner I und Reaktor IV, geführt wird.

Typischerweise handelt es sich bei den wässrigen Phasen, z.B. P2 und P4, um die schwerere Phase und bei den organischen Phasen, z.B. P1 und P3 um die leichtere Phase der jeweiligen Trennung.

Die bevorzugten Schritte der Aufarbeitung der Reaktionsmischung aus Reaktor II werden im Folgenden näher beschrieben.

### Destillationsstufe I (Schritt i)

Bevorzugt umfasst das erfindungsgemäße Verfahren das Trennen der Reaktionsmischung aus der oxidativen Veresterung aus Reaktor II in einer Destillationsstufe I, insbesondere in einer Destillationskolonne I, wobei Methacrolein und teilweise Alkohol entfernt und zum Reaktor II zurückgeführt werden, und wobei ein Strom W1, enthaltend Alkylmethacrylat, Methacrylsäure und/oder deren Salze, Alkohol und Wasser, erhalten wird.

In einer bevorzugten Ausführungsform des erfindungsgemäßen Verfahrens wird das Methacrolein nicht oder nur teilweise direkt in den Reaktor II geführt, sondern über die Destillationsstufe I in das Verfahren eingeführt. Bevorzugt wird Methacrolein vollständig oder teilweise in die Destillationsstufe I zugegeben und über die niedrigsiedende Kopf-Fraktion des Destillationsstufe I zum Reaktor II geführt.

### Reaktor III - Acetal-Spalter (optionaler Schritt ii)

Das erfindungsgemäße Verfahren umfasst bevorzugt das Einleiten der Sumpf-Fraktion W1 aus Destillationsstufe I in einen Reaktor III (Acetal-Spalter) und Zugabe mindestens einer Säure S, wobei im Strom W1 enthaltende Acetale gespalten werden, und wobei ein Strom W2 erhalten wird.

Bevorzugt wird in Reaktor III das Acetal-Nebenprodukt Dimethoxyisobuten (DMIB) zu Methacrolein (MAL) und Methanol (MeOH) gespalten. Eine Acetalspaltung ist beispielsweise in WO 2019/042807 und JP 11-302224A beschrieben. Insbesondere kann durch die Entfernung der Acetal-Nebenprodukte im optionalen Verfahrensschritt ii) die Verfärbung in Folgeprodukte des Alkylmethacrylats, wie Polymeren und Formmassen, reduziert werden.

Typischerweise werden zudem in Reaktor III Salze der Methacrylsäure, wie insbesondere Alkalimethacrylate, neutralisiert, wobei freie Methacrylsäure gebildet wird.

Typischerweise erfolgt im Reaktor III eine Vermischung der Sumpf-Fraktion W1 mit mindestens einer Säure S und optional Wasser. Reaktor III kann in einer dem Fachmann bekannten Art ausgestaltet sein, beispielsweise als Rohrreaktor bevorzugt mit einem statischen Mischer ausgerüstet, als Rührreaktor oder als Kombination hiervon.

Bevorzugt umfasst Reaktor III einen Säure-Zulauf I, wobei mindestens eine Säure S, bevorzugt Schwefelsäure, zugeführt wird. Es ist zudem möglich, dass die mindestens eine Säure S (ausschließlich oder teilweise) über die Rückführung der Sumpf-Fraktion W4 aus der Destillationsstufe II zugeführt wird. Optional kann Reaktor III zusätzlich zum Säure-Zulauf I einen Wasserzulauf umfassen.

Bevorzugt liegt der pH-Wert im Reaktor III im Bereich von 0 bis 7,0; bevorzugt 0,5 bis 5,0.

Typischerweise wird die Zugabe der mindestens einen Säure S (bevorzugt Schwefelsäure) in Reaktor III so gewählt, dass die wässrige Phase P2 in der Extraktion ein pH-Wert im Bereich von kleiner oder gleich pH 4 aufweist, bevorzugt ph 1,5 bis pH 3.

In einer bevorzugten Ausführungsform ist der optionale Verfahrensschritt ii) verwirklicht und die Zugabe an Säure S (bevorzugt Schwefelsäure) in Reaktor III, insbesondere die Zugabe der Säure S über den Säure-Zulauf I, wird so gewählt, dass sich im kontinuierlichen Betrieb ein pH Wert im Bereich von 0 bis 3, bevorzugt von 2, in der Sumpf-Fraktion W4 aus Destillationsstufe II einstellt.

In einer bevorzugten Ausführungsform ist der optionale Verfahrensschritt ii) verwirklicht und die Zugabe an frischer Säure S (bevorzugt Schwefelsäure) in das erfindungsgemäße Verfahren erfolgt ausschließlich über die Zugabe in Reaktor III, bevorzugt über den Säure-Zulauf I.

### Extraktion (Schritt iii)

Das erfindungsgemäße Verfahren umfasst mindestens eine Extraktion.

Bevorzugt umfasst das Verfahren die Extraktion des Stroms W1 (ohne optionalen Schritt ii)) oder W2 (mit optionalem Schritt ii)) mit Wasser und Auftrennen in eine organische Phase P1 (typischerweise die leichte Phase), enthaltend Alkylmethacrylat und Methacrylsäure, und eine wässrige Phase P2 (typischerweise die schwere Phase), enthaltend Wasser, Säure S und/oder deren Salze, Alkohol, Methacrylsäure und/oder deren Salze. Bevorzugt erfolgt die Zugabe von Wasser im oberen Teil der Kolonne.

Typischerweise enthält die organische Phase P1 hauptsächlich das Alkylmethacrylat und organischen Nebenprodukte der Umsetzung, wie Methacrylsäure und weitere schwersiedenden Nebenprodukte, und kleinere Mengen an Wasser und Methanol. Typischerweise enthält die wässrige Phase P2 wenig organische Produkte und enthält hauptsächlich Wasser und Methanol sowie Alkali- oder Erdalkalisalz aus der Neutralisation und Salze der Methacrylsäure, insbesondere Alkalisalze der Methacrylsäure.

Bevorzugt wird bei der Extraktion mindestens eine Säure S zugegeben. Bevorzugt wird die Zugabe der mindestens einen Säure S (bevorzugt Schwefelsäure) in der Extraktion, so gewählt, dass die wässrige Phase P2 in der Extraktion ein pH-Wert im Bereich von größer oder gleich 3 aufweist. Typischerweise werden durch die Zugabe der Säure S in der Extraktion Salze der Methacrylsäure, wie insbesondere Alkalimethacrylate, neutralisiert.

Optional kann der Extraktion Wasser über einen optionalen Zulauf II zugeführt werden. Optional kann der Extraktion Säure S, z.B. über einen optionalen Säure-Zulauf und/oder zusammen mit dem Wasser-Zulauf II, zugegeben werden. Es ist zudem möglich, dass das Wasser und optional die Säure S der Extraktion (ausschließlich oder teilweise) über die Rückführung der Sumpf-Fraktion W4 aus der Destillationsstufe II zugeführt wird.

Bevorzugt wird die Extraktion in Schritt iii) in einer Extraktionskolonne durchgeführt. Dem Fachmann sind typische Ausführungsformen von Extraktionskolonne bekannt. Typischerweise können Bodenkolonne oder gepackte Kolonnen, wie beispielsweise Füllkörperkolonnen (z.B. mit Raschig-Ringen) oder Kolonnen mit regelmäßigem Packmaterial (z.B. Mellapak von Sulzer) eingesetzt werden.

In einer bevorzugten Ausführungsform erfolgt die Zugabe von Wasser zur Extraktion über den Wasserzulauf II im oberen Bereich einer Destillationskolonne, bevorzugt zu der ersten Extraktionsstufe, bevorzugt oberhalb einer möglichen Rückführung der Sumpf-Fraktion W4 aus der Destillationsstufe II.

In einer bevorzugten Ausführungsform wird die Extraktion (Schritt iii) in einer Extraktionskolonne durchgeführt und die Sumpf-Fraktion W4 aus Destillationsstufe II wird im mittleren Bereich der Extraktionskolonne, bevorzugt unterhalb der ersten Extraktionsstufe, besonders bevorzugt unter der zweiten Extraktionsstufe, zugegeben. Weiterhin bevorzugt erfolgt die Zugabe von Wasser und optional Säure S zu der Extraktion in der Extraktionskolonne oberhalb von der Zugabe der Sumpf-Fraktion W4 aus Destillationsstufe II.

Die organische Phase P1 der Extraktion, welche den größten Anteil des gewünschten Alkylmethacrylat-Produktes enthält, wird bevorzugt über die im Folgenden beschriebenen Destillationsstufen III und IV und optional V und VI weiter gereinigt. Hierbei werden der organischen Phase P1, welche den überwiegenden Anteil des Alkylmethacrylats enthält, zunächst schwerersiedende Komponenten (Destillationsstufe III) und dann leichter-siedende Komponenten (Destillationsstufe IV) entnommen.

Die wässrige Phase W4 enthält typischerweise Mineralsalze der entsprechenden Brönstedtsäure, wobei der Salzgehalt im Bereich von 0,5 bis 15 Gew%, bezogen auf W4 beträgt. Die Rückführung der salzhaltigen Phase W4 in die Extraktion bewirkt typischerweise eine Verbesserung der Extraktionsleistung. In der Regel beruht dieser Effekt auf eine gegenüber VE Wasser erhöhte Dichte und damit einer höheren Dichtedifferenz gegenüber der organischen Phase.

### Destillationsstufe II - Alkohol-Rückgewinnung (Schritt iv)

Bevorzugt umfasst das erfindungsgemäße Verfahren das Trennen der wässrigen Phase P2 aus der Extraktion in einer Destillationsstufe II (bevorzugt in einer Destillationskolonne II), wobei eine niedrigsiedende Kopf-Fraktion W3, enthaltend hauptsächlich Alkohol, und eine schwersiedende Sumpf-Fraktion W4, enthaltend Wasser, Säure S und/oder deren Salze, und Methacrylsäure und/oder deren Salze, erhalten werden.

Bevorzugt wird die Kopf-Fraktion W3 vollständig oder teilweise in den Reaktor II zurückgeführt.

Erfindungsgemäß wird die wässrige Sumpf-Fraktion aus der Destillationsstufe II an eine oder mehrere verschiedene Stellen des erfindungsgemäßen Verfahrens zurückgeführt, wodurch Wasser und/oder Säure S eingespart werden und die Menge an wässrigen Abfallströmen reduziert wird.

Erfindungsgemäß wird die Sumpf-Fraktion W4 aus Destillationsstufe II vollständig oder teilweise in einen oder mehrere der Verfahrensteile, ausgewählt aus Reaktor III, Extraktion, Phasentrenner I und Reaktor IV, geführt.

In einer bevorzugten Ausführungsform des Verfahrens wird ein Teil der Sumpf-Fraktion W4 aus Destillationsstufe II kontinuierlich und/oder diskontinuierlich (als Purge) aus dem Verfahren ausgeschleust wird.

Bevorzugt wird die Sumpf-Fraktion W4 aus Destillationsstufe II vollständig oder teilweise in die Extraktion (Schritt iii) zurückgeführt wird. Bevorzugte Ausführungsformen der Zugabe von W4 zur Extraktion sind oben beschrieben.

Bevorzugt wird die Sumpf-Fraktion W4 aus Destillationsstufe II vollständig oder teilweise in den Phasentrenner I (Schritt vii) geführt.

In einer bevorzugten Ausführungsform ist der optionale Verfahrensschritt ii) (Acetal-Spalter) verwirklicht und die Sumpf-Fraktion W4 aus Destillationsstufe II wird vollständig oder teilweise in den Reaktor III (Acetal-Spalter) zurückgeführt.

In einer bevorzugten Ausführungsform ist der optionale Verfahrensschritt viii) verwirklicht und die Sumpf-Fraktion W4 aus Destillationsstufe II wird vollständig oder teilweise in den Reaktor IV (Esterhydrolyse) geführt.

In einer bevorzugten Ausführungsform erfolgt die Rückführung der Sumpf-Fraktion W4 in zwei oder mehr der genannten Verfahrensteile.

Bevorzugt weist die wässrige Sumpf-Fraktion W4 einen pH-Wert im Bereich von 1 bis 3, bevorzugt 1,5 bis 2,5 auf. Bevorzugt enthält die wässrige Sumpf-Fraktion W4 mehr als 60 Gew.-%, bevorzugt mehr als 80 Gew.-%, bezogen auf das Gesamtgewicht W4, Wasser. Bevorzugt enthält die Sumpf-Fraktion W4 weniger als 10 Gew.-%, bevorzugt weniger als 5 Gew.-%, besonders bevorzugt weniger als 1 Gew.-%, bezogen auf das Gesamtgewicht W4, Alkohol insbesondere Methanol.

### Destillationsstufe III - Abtrennung der Schwersieder (Schritt v)

Bevorzugt umfasst das erfindungsgemäße Verfahren das Trennen der organischen Phase P1 aus der Extraktion in einer Destillationsstufe III (insbesondere in einer Destillationskolonne III), wobei eine niedrigsiedende Kopf-Fraktion W5, enthaltend Alkylmethacrylat, und eine schwersiedende Sumpf-Fraktion W6, enthaltend Methacrylsäure, erhalten werden.

In einer Ausführungsform kann ein Teil der Kopf-Fraktion W5 in einen Phasentrenner II geführt werden. Die wässrige Phase kann Reaktor IV zugeführt werden.

In einer bevorzugten Ausführungsform kann die Sumpf-Fraktion W6, enthaltend Methacrylsäure, in eine optionale Destillationsstufe VI (Destillationskolonne VI) geführt werden, wobei die Menge an Alkylmethacrylat in W6 reduziert wird und die Kopf-Fraktion der Destillationsstufe VI, enthaltend Alkylmethacrylat, in die Destillationsstufe III zurückgeführt werden kann.

In einer bevorzugten Ausführungsform kann aus der Sumpf-Fraktion W6 und/oder aus der Sumpf-Fraktion der optionalen Destillationsstufe VI Methacrylsäure als weiteres Produkt gewonnen werden. Einzelheiten hierzu sind beispielweise in WO 2017/046110 beschreiben
Alternativ können auch eine, mehrere oder alle Destillationsstufen III, IV und V zur Aufreinigung des Alkylmethacrylats durch eine Kristallisation ersetzt werden.

### Destillationsstufe IV - Abtrennung der Leichtsieder (Schritt vi)

Bevorzugt umfasst das erfindungsgemäße Verfahren das Trennen der niedrigsiedende Kopf-Fraktion W5 aus Destillationsstufe III in einer Destillationsstufe IV (insbesondere in einer Destillationskolonne IV), wobei eine niedrigsiedende Kopf-Fraktion W7 und eine schwersiedende Sumpf-Fraktion W8, enthaltend Alkylmethacrylat, erhalten werden.

Bevorzugt kann die Sumpf-Fraktion W8 in eine weitere optionale Destillationsstufe V (insbesondere Destillationskolonne V) geführt werden, wobei eine Endreinigung des Alkylmethacrylats erfolgt und das Alkylmethacrylat als Kopf-Fraktion der optionalen Destillationsstufe V als Produkt aus dem Verfahren ausgeschleust wird.

Die Sumpf-Fraktion der optionalen Destillationsstufe V kann optional in die Destillationsstufe III und/oder die Destillationsstufe VI geführt werden.

### Phasentrenner I (Schritt vii)

Bevorzugt umfasst das erfindungsgemäße Verfahren das Vermischen der Kopf-Fraktion W7 aus der Destillationsstufe IV mit Wasser und Auftrennen in eine organische Phase P3 (typischerweise die leichte Phase) und eine wässrige Phase P4 (typischerweise die schwere Phase) in einem Phasentrenner I.

Typische Ausgestaltungen von Phasentrennern sind dem Fachmann bekannt.

Bevorzugt kann die organische Phase P3 aus dem Phasentrenner I vollständig oder teilweise, kontinuierlich oder diskontinuierlich aus dem Verfahren als organischer Abfallstrom ausgeschleust werden. Bevorzugt wird die organische Phase P3 aus dem Phasentrenner I vollständig oder teilweise in eine optionale Destillationsstufe VII (MAL-Rückgewinnung) geführt, in welcher Methacrolein zurückgewonnen und der oxidativen Veresterung in Reaktor II zugeführt wird.

In einer alternativen Ausführungsform wird die wässrige Phase P4 aus dem Phasentrenner I vollständig oder teilweise in die Destillationskolonne II (Alkohol-Rückgewinnung) geführt. Dies erfolgt insbesondere, wenn der optionale Verfahrensschritt viii) nicht verwirklicht ist.

Es ist auch möglich, die wässrige Phase P4 aus Phasentrenner I vollständig oder teilweise, kontinuierlich und/oder diskontinuierlich als wässrigen Abwasserstrom auszuschleusen.

Bevorzugt umfasst der Phasentrenner I einen Wasser-Zulauf III. Es ist zudem möglich, dass das Wasser (ausschließlich oder teilweise) über die Rückführung der Sumpf-Fraktion W4 aus der Destillationsstufe II zugeführt wird.

### Reaktor IV - Esterhydrolyse (optionaler Schritt viii)

Bevorzugt umfasst das erfindungsgemäße Verfahren das Vermischen der wässrigen Phase P4 aus Phasentrenner I mit mindestens einer Säure S in Reaktor IV (Esterhydrolyse), wobei in P4 enthaltende Ester-Nebenprodukte gespalten werden und Alkohol zurückgewonnen wird, und wobei eine wässrige Phase P5 erhalten wird.

Typischerweise werden im optionalen Reaktor IV Ester-Nebenprodukten, welche einen niedrigeren Siedepunkt als Methylmethacrylat aufweisen, insbesondere gesättigte Ester wie Isobuttersäurealkylester und Propionsäurealkylester, beispielsweise Isobuttersäure-methylester, Propionsäuremethylester, hydrolysiert, wobei Alkohol, bevorzugt Methanol, zurückgewonnen werden kann.

In einer bevorzugten Ausführungsform des Verfahrens ist der optionale Verfahrensschritt viii) verwirklicht und die wässrige Phase P5 aus Reaktor IV (Esterhydrolyse) wird vollständig oder teilweise in die Destillationskolonne II geführt. Hierbei wird typischerweise der in der Esterhydrolyse zurückgewonnene Alkohol über die Kopf-Fraktion W3 dem Reaktor II zugeführt.

Es ist auch möglich, einen Teil der wässrige Phase P5 aus Reaktor IV kontinuierlich und/oder diskontinuierlich als wässrigen Abwasserstrom auszuschleusen.

Bevorzugt umfasst Reaktor IV einen Säure-Zulauf IV, wobei mindestens eine Säure S, bevorzugt Schwefelsäure, zugeführt wird. Es ist zudem möglich, dass die mindestens eine Säure S (ausschließlich oder teilweise) über die Rückführung der Sumpf-Fraktion W4 aus der Destillationsstufe II zugeführt wird. Optional kann Reaktor IV zusätzlich zum Säure-Zulauf IV einen Wasserzulauf umfassen.

### Beschreibung der Figur und Bezugszeichenliste

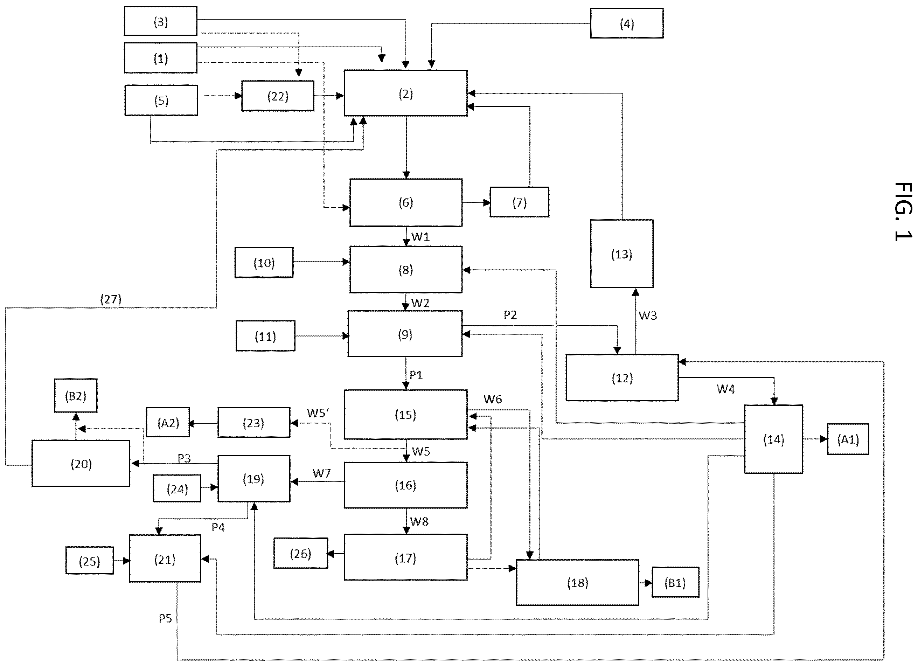

Figur 1 zeigt beispielhaft ein mögliches schematisches Fließbild des zweiten Verfahrensschritts (oxidative Veresterung MAL und Aufarbeitung des Produktstroms) des erfindungsgemäßen Verfahrens zur Herstellung von Alkylmethacrylat. Reaktor I des erfindungsgemäßen Verfahrens zur MAL-Synthese ist nicht abgebildet.
- (1): Zulauf MAL in Reaktor II
- (2): Reaktor II zur oxidativen Veresterung des MAL
- (3): Zulauf Alkohol (insbesondere Methanol) in Reaktor II
- (4): Sauerstoff- und/oder Luft-Zuleitung in Reaktor II
- (5): Zulauf Base in Reaktor II
- (6): Destillationskolonne I zur Abtrennung MAL
- (7): Niedrigsiedende Fraktion enthaltend MAL und Alkohol zur Rückführung in Reaktor II (Recyclingstrom)
- (8): Reaktor III (Acetal-Spalter) zur Spaltung von Acetal-Nebenprodukten, (z.B. Dimethoxyisobuten (DMIB) zu MAL und MeOH) (optional)
- (9): Extraktion
- (10): Optionaler Säure-Zulauf I zu (8)
- (11): Optionaler Wasser-Zulauf II zu (9)
- (12): Destillationskolonne II zur Rückgewinnung von Alkohol
- (13): Niedrigsiedende Fraktion der Destillationskolonne II enthaltend Alkohol zur Rückführung in Reaktor II
- (14): Sumpffraktion der Destillationskolonne II, enthaltend Wasser, Säure und Alkylmethacrylsäure und/oder deren Salze
- (15): Destillationskolonne III zur Abtrennung von Schwersiedern
- (16): Destillationskolonne IV zur Abtrennung von Leichtsiedern
- (17): Destillationskolonne V zur Endreinigung des MMA
- (18): Destillationskolonne VI zur Reduzierung der Menge von Alkylmethacrylat im Sumpfstrom von (15) (optional)
- (19): Phasentrenner I zur Aufarbeitung der niedrigsiedenden Fraktion aus Kolonne IV (16)
- (20): Destillationskolonne VII zur Rückgewinnung von MAL aus organischer Phase aus Phasentrenner I (19) (optional)
- (21): Reaktor IV (Ester-Spalter) zur Rückgewinnung von Alkohol aus Ester-Nebenprodukten (z.B gesättigten Estern wie Isobuttersäurealkylester, Propionsäurealkylester) (optional)
- (22): Vorrichtung zum Mischen der Edukte Mischer I (optional)
- (23): Wasseraufbereitung (optional)
- (24): Wasser-Zulauf III zu (19) (optional)
- (25): Säure-Zulauf IV zu (21) (optional)
- (26): Alkylmethacrylat-Produktstrom
- (27): Recyclingstrom enthaltend MAL
- (A1)/(A2): wässriger Abfallstrom
- (B1)/(B2): organischer Abfallstrom

### Experimenteller Teil

### Beispiel 1 - mit Rückführung W4 in die Extraktion (9)

In Reaktor II erfolgte die Umsetzung von Methacrolein mit Methanol in Gegenwart eines sauerstoffhaltigen Gases in flüssiger Phase zu Methylmethacrylat (MMA). Der Reaktoraustrag aus Reaktor II wies die folgende Zusammensetzung auf: MEOH 43,1 Gew%, MAL 8,8 Gew%, MMA 37,0 Gew%, H2O 6,6 Gew%, MAL-Acetal 360 ppm, Rest 4,4 Gew%.

Der Austrag aus Reaktor II (2) wurde direkt der Extraktion (9) zugeführt. Die Extraktion wurde in einer Extraktionskolonne durchgeführt. Teilweise erfolgte ein Wasser-Zulauf II (11) zur Extraktion. Die wässrige Phase P2 der Extraktion wurde in Kolonne II (12) geführt. Es wurde ein wässriger Abwasserstrom A1 aus dem Sumpfstrom der Kolonne II ausgeschleust. Teilweise erfolgte eine Rückführung des Sumpfstroms der Kolonne II (W4) zur Extraktion (9).

Im Fall von Versuch 3 erfolgte die Zugabe von W4 und Wasser am Kopf der Extraktionskolonne (9). Im Fall von Versuch 4 erfolgte die Zugabe von W4 unterhalb des Kopfes der Extraktionskolonne und die Zugabe an Wasser erfolgte am Kopf der Extraktionskolonne (9).

Die Aufarbeitung der organischen Phase der Extraktion erfolgte über die Kolonnen III (15), IV (16) und V (17). In der Kolonne V wurde der MMA-Produktstrom (26) als Kopf-Fraktion der entnommen.

Die Zuläufe in die Extraktion (9) und die Menge an wässrigen Abfallströmen sind in der nachfolgenden Tabelle 1 zusammengefasst.

**Tabelle 1: Zusammenfassung der Versuche 1-4**

| V-Nr. | Zuläufe Extraktion | | Abwasserstrom A1 | Produktstrom MMA | Gehalt Methanol und MMA in W4 | pH-Wert W4 |
|---|---|---|---|---|---|---|
| | Wasser-Zulauf II | W4 | | | | |
| | kg/h | kg/h | kg/h | kg/h | Gew.-% | |
| 1 (Ref) | 24,1 Wasser 0,50 Säure | / | 29,2 W4 vollständig | 15,65 | MEOH 500 ppm MMA 0 | 2 |
| 2 | 0 Wasser 0,19 Säure | 24,1 (75% von W4) | 6,5 (25% von W4) | 15,65 | MEOH 500 ppm MMA 0 | 2 |
| 3 | 8,8 Wasser 0,30 Säure | 15,3 (50 % von W4) | 15,3 (50% von W4) | 15,65 | MEOH 500 ppm MMA 0 | 2 |
| 4 | 8,8 Wasser 0,30 Säure (Kopf) | 15,3 (50 % von W4) (unterhalb Kopf) | 15,3 (50% von W4) | 15,65 | MEOH 500 ppm MMA 0 | 2 |

### Beispiel 2 - mit Rückführung W4 in Reaktor III /Acetal-Spalter

In Reaktor II erfolgte die Umsetzung von Methacrolein mit Methanol in Gegenwart eines sauerstoffhaltigen Gases in flüssiger Phase zu Methylmethacrylat (MMA). Der Reaktoraustrag aus Reaktor II wies die folgende Zusammensetzung auf: MEOH 43,1 Gew%, MAL 8,8 Gew%, MMA 37,0 Gew%, H2O 6,6 Gew%, MAL-Acetal 360 ppm, Rest 4,4 Gew%..

Der Austrag aus Reaktor II (2) wurde zur Aufarbeitung in den Reaktor III (8) geführt. Reaktor III war als kontinuierlich betriebener Rührkessel mit nachgeschaltetem Dekanter ausgeführt. Teilweise erfolgte ein Zulauf an 96%-iger Schwefelsäure (Säure S) über Säure-Zulauf I (10) zu Reaktor III. Der Austrag aus Reaktor III wurde der Extraktion (9) zugeführt.

Die Extraktion wurde in einer Extraktionskolonne durchgeführt. Teilweise erfolgte ein Wasser-Zulauf II (11) zur Extraktion. Die wässrige Phase P2 der Extraktion wurde in Kolonne II (12) geführt. Es wurde ein wässriger Abwasserstrom A1 aus dem Sumpfstrom der Kolonne II ausgeschleust. Teilweise erfolgte eine Rückführung des Sumpfstroms der Kolonne II (W4) zum Reaktor III und/oder zur Extraktion (9).

Die Aufarbeitung der organischen Phase der Extraktion erfolgte über die Kolonnen III (15), IV (16) und V (17). In der Kolonne V wurde der MMA-Produktstrom (26) als Kopf-Fraktion der entnommen.

Die Zuläufe in Reaktor III und Extraktion sind in der nachfolgenden Tabelle 2 zusammengefasst.

**Tabelle 2: Zusammenfassung der Versuche 5-7**

| V-Nr | Zuläufe Reaktor III | | Zuläufe Extraktion | | Abwasser A1 | MAL-Acetal Gehalt in W2 | Produktstrom MMA | Gehalt an Methanol und MMA in W4 | pH-Wert W4 |
|---|---|---|---|---|---|---|---|---|---|
| | Zulauf I Wasser & Säure | W4 | Zulauf II Wasser | W4 | | | | | |
| | kg/h | kg/h | kg/h | kg/h | kg/h | ppm | kg/h | Gew.-% | |
| 5 (Ref) | 21,0 Wasser 0,50 Säure | / | 3,1 | / | 29,2 (W4 vollständi g) | 14 | 15,65 | MEOH 500 ppm MMA 0 | 2 |
| 6 | 0 Wasser 0,23 Säure | 21,0 Wasser (69% von W4) | 3,1 | / | 9,6 (31 % von W4) | 14 | 15,65 | MEOH 500 ppm MMA 0 | 2 |
| 7 | 0 Wasser 0,19 Säure | 21,0 (69% von W4) | 0 | 3,1 (10% von W4) | 6,5 (21 % von W4) | 14 | 15,65 | MEOH 500 ppm MMA 0 | 2 |

### Beispiel 3 - mit Rückführung W4 in Phasentrenner I

Die Durchführung erfolgte wie in Beispiel 1 beschrieben. Die Kopf-Fraktion aus der Kolonne IV wurde mit über den Wasser-Zulauf III (24) mit Wasser versetzt und danach im Phasentrenner **I** (19) in eine organische Phase P3 und in eine wässrige Phase P4 aufgetrennt. Die wässrige Phase P4 wurde in die Kolonne II (12) zurückgeführt.

Teilweise erfolgte eine Rückführung des Sumpfstroms der Kolonne II (W4) zum Phasentrenner **I** und/oder zur Extraktion. Die Zuläufe in Phasentrenner **I** und Extraktion sind in der nachfolgenden Tabelle 3 zusammengefasst.

**Tabelle 3: Zusammenfassung der Versuche 8-10**

| V-Nr | Zuläufe Phasentrenner **I** | | Zuläufe Extraktion | | Abwasser A1 | Produktstrom MMA | Gehalt an Methanol und MMA in W4 | pH-Wert W4 |
|---|---|---|---|---|---|---|---|---|
| | Zulauf III Wasser | W4 | Zulauf II Wasser und Säure | W4 | | | | |
| | kg/h | kg/h | kg/h | kg/h | kg/h | kg/h | Gew.-% | |
| 8 (Ref) | 2,5 | / | 24,1 Wasser 0,5 Säure | / | 29,2 (W4 vollständig) | 15,65 | MEOH 500 ppm MMA 0 | 2 |
| 9 | 0 | 2,5 (9% von W4) | 24,1 Wasser 0,5 Säure | / | 26,7 (91 % von W4) | 15,65 | MEOH 500 ppm MMA 0 | 2 |
| 10 | 0 | 2,5 (8% von W4) | 0 Wasser 0,19 Säure | 24,1 (78% von W4) | 4,0 (14% von W4) | 15,65 | MEOH 500 ppm MMA 0 | 2 |

### Beispiel 4 - mit Rückführung W4 in Reaktor IV / Esterspalter

Die Durchführung erfolgte wie in Beispiel 3 beschrieben. Der Phasentrenner **I** wurde über Wasser-Zulauf III (24) mit Wasser versorgt. Die wässrige Phase P4 aus Phasentrenner **I** (19) wurde in den Reaktor IV (Ester-Spalter) (21) geführt und über Säure-Zulauf IV mit einer 96%-iger Schwefelsäure (Säure S) vermischt. Die wässrige Phase P5 aus Reaktor IV wurde in Kolonne II (12) zurückgeführt.

Teilweise erfolgte eine Rückführung des Sumpfstroms der Kolonne II (W4) zum Reaktor IV und/oder zur Extraktion. Die Zuläufe in den Reaktor IV und Extraktion sind in der nachfolgenden Tabelle 4 zusammengefasst.

**Tabelle 4: Zusammenfassung der Versuche 11-13**

| V-Nr | Zuläufe Reaktor IV | | Zuläufe Extraktion | | Abwasser A1 | Produktstrom MMA | Gehalt an Methanol und MMA in W4 | pH-Wert W4 |
|---|---|---|---|---|---|---|---|---|
| | Zulauf IV Säure | W4 | Zulauf II Wasser und Säure | W4 | | | | |
| | kg/h | kg/h | kg/h | kg/h | kg/h | kg/h | Gew.-% | |
| 11 (Ref) | 0,016 | / | 24,1 Wasser 0,5 Säure | / | 29,2 (W4 vollständig) | 15,65 | MEOH 500 ppm MMA 0 | 2 |
| 12 | 0 | 1,4 (5% von W4) | 24,1 Wasser 0,5 Säure | / | 29,2 (95% von W4) | 15,65 | MEOH 500 ppm MMA 0 | 2 |
| 13 | 0 | 1,4 (4% von W4) | 0 Wasser 0,19 Säure | 24,1 (75% von W4) | 6,5 (21 % von W4) | 15,65 | MEOH 500 ppm MMA 0 | 2 |

Es zeigt sich, dass durch die erfindungsgemäße Verfahrensführung eine Einsparung an Wasser- und/oder Säure-Zuläufen und eine Minimierung des wässrigen Abwasserstroms A1 bei gleichbleibender Produktqualität und Ausbeute möglich sind.

## Patentansprüche

1. Verfahren zur Herstellung von Alkylmethacrylaten, bei dem in einer ersten Reaktionsstufe in einem Reaktor **I** Methacrolein hergestellt und dieses in einer zweiten Reaktionsstufe in einem Reaktor II oxidativ mit einem Alkohol in Gegenwart eines sauerstoffhaltigen Gases unter Entstehung von Reaktionswasser in flüssiger Phase zu einem Alkylmethacrylat verestert wird, **dadurch gekennzeichnet, dass**
a. die Aufarbeitung der Reaktionsmischung aus Reaktor II zu Alkylmethacrylat mindestens eine Destillation und mindestens eine Extraktion umfasst;
b. eine wässrige, Alkohol und Alkali- und/oder Erdalkalisalz einer Brönstedt-Säure enthaltende Phase der Extraktion in mindestens einer Destillation in einer Kolonne II derart behandelt wird, dass im Sumpf der Kolonne II ein Prozesswasserstrom enthaltend Reaktionswasser und Alkali- und/oder Erdalkalisalz einer Brönstedt-Säure entsteht, wobei in diesem Prozesswasserstrom der Gehalt an Alkohol und Alkylmethacrylat kleiner 5 Gew.-%, bezogen auf den gesamten Prozesswasserstrom, beträgt, und
c. dieser Prozesswasserstrom aus dem Sumpf der Kolonne II teilweise aus dem Verfahren ausgeschleust und einer Entsorgung zugeführt wird und teilweise in die Aufarbeitung der Reaktionsmischung aus Reaktor II zurückgeführt wird.

2. Verfahren gemäß Anspruch 1, **dadurch gekennzeichnet, dass** der Prozesswasserstrom aus dem Sumpf der Kolonne II teilweise in die Extraktion zurückgeführt wird, wobei dieser zurückgeführte Prozesswasserstrom in der Extraktion mit einer Alkylmethacrylat und Alkohol enthaltenden organischen Phase in Kontakt gebracht wird.

3. Verfahren gemäß Anspruch 1 oder 2, **dadurch gekennzeichnet, dass** der Prozesswasserstrom aus dem Sumpf der Kolonne II teilweise in einen Reaktor III zurückgeführt wird, wobei dieser zurückgeführte Prozesswasserstrom in Reaktor III mit einer Alkylmethacrylat und Alkohol enthaltenden organischen Phase, welche einen Gehalt an Methacrolein-Acetal von kleiner 3 Gew.-%, bezogen auf die organischen Phase, aufweist, in Kontakt gebracht wird.

4. Verfahren gemäß einem der Ansprüche 1 bis 3, **dadurch gekennzeichnet, dass** die Extraktion in einer Extraktionskolonne und/oder in einer in Reihe geschalteten Serie von mindestens zwei Mixer-Settler-Apparaten durchgeführt wird und der Prozesswasserstrom aus dem Sumpf der Kolonne II unterhalb des Kopfes der Extraktionskolonne oder im Mixerbereich eines Mixer-Settler-Apparates zugegeben wird, und optional im Kopfbereich der Extraktionskolonne Wasser, insbesondere vollentsalztes Wasser, zugegeben wird.

5. Verfahren gemäß Anspruch 4, **dadurch gekennzeichnet, dass** die Zugabe von Wasser und optional einer Brönstedt-Säure zu der Extraktion oberhalb von der Zugabe des Prozesswasserstroms aus dem Sumpf der Kolonne II erfolgt.

6. Verfahren gemäß Anspruch 3, **dadurch gekennzeichnet, dass** in Reaktor III die Zugabe einer Brönstedt-Säure erfolgt und diese Zugabe so gewählt wird, dass sich im kontinuierlichen Betrieb ein pH Wert im Bereich von 1,5 bis 2,5 in dem Prozesswasserstrom aus dem Sumpf der Kolonne II einstellt.

7. Verfahren gemäß Anspruch 6, **dadurch gekennzeichnet, dass** die Zugabe einer Brönstedt-Säure in das erfindungsgemäße Verfahren ausschließlich über die Zugabe in Reaktor III erfolgt.

8. Verfahren gemäß einem der Ansprüche 1 bis 7, **dadurch gekennzeichnet, dass** in der Extraktion eine organische Phase enthaltend Alkylmethacrylat und Methacrylsäure erhalten wird und die organische Phase aus der Extraktion in einer Destillationsstufe IV in eine Sumpf-Fraktion enthaltend Alkylmethacrylat und eine leichter-siedende Kopf-Fraktion getrennt wird.

9. Verfahren gemäß Anspruch 8, **dadurch gekennzeichnet, dass** die Kopf-Fraktion aus der Destillationsstufe IV mit Wasser versetzt wird und danach in einem Phasentrenner **I** in eine organische Phase und in eine wässrige Phase aufgetrennt wird.

10. Verfahren gemäß Anspruch 9, **dadurch gekennzeichnet, dass** die wässrige Phase aus dem Phasentrenner **I** mit mindestens einer Brönstedt-Säure in einem Reaktor IV vermischt wird, wobei in der wässrige Phase aus dem Phasentrenner **I** enthaltende Ester-Nebenprodukte gespalten werden und Alkohol zurückgewonnen wird, und optional der Prozesswasserstrom aus dem Sumpf der Kolonne II teilweise in den Reaktor IV geführt wird.

11. Verfahren gemäß Anspruch 10, **dadurch gekennzeichnet, dass** das Produkt aus dem Reaktor IV vollständig oder teilweise in die Kolonne II geführt wird.

12. Verfahren gemäß Anspruch 9, **dadurch gekennzeichnet, dass** die wässrige Phase aus Phasentrenner **I** vollständig oder teilweise in die Kolonne II geführt wird.

13. Verfahren gemäß einem der Ansprüche 1 bis 12, **dadurch gekennzeichnet, dass** die Reaktionsmischung aus Reaktor II in einer Destillationsstufe **I** getrennt wird, wobei Methacrolein und teilweise Alkohol über die Kopf-Fraktion entfernt und zum Reaktor II zurückgeführt werden.

14. Verfahren gemäß Anspruch 13, **dadurch gekennzeichnet, dass** Methacrolein vollständig oder teilweise in die Destillationsstufe **I** zugegeben wird und über die Kopf-Fraktion der Destillationsstufe **I** zum Reaktor II geführt wird.

15. Verfahren gemäß einem der Ansprüche 1 bis 14, **dadurch gekennzeichnet, dass** der Alkohol Methanol ist und das Alkylmethacrylat Methylmethacrylat ist.

16. Verfahren gemäß einem der Ansprüche 1 bis 15, **dadurch gekennzeichnet, dass** die Brönstedt-Säure Schwefelsäure ist.
